# EUROPEAN PATENT SPECIFICATION

(11) **EP 1 933 141 B1**
(45) Date of publication and mention of the grant of the patent: **17.08.2011**
(21) Application number: 06025524.7
(22) Date of filing: 11.12.2006
(51) Int. Cl.: G01N 33/558, G01N 33/543, G01N 33/58, C12Q 1/68

(54) **Antigen detection method involving an oligonucleotide enhanced collodial gold signal**
Verfahren zur Detektion von Antigenen mittels eines durch Oligonukleotide verstärkten kolloidalen Gold Signals
Procédé pour la détection des antigènes par un signal d'or colloïdal amplifié par des oligonucléotides

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- WO-A-02/46472
- ANONYMOUS: "QuickStepTM HCG - One Step Urine Pregnancy Test" INTERNET ARTICLE, [Online] March 1998 (1998-03), XP002423569 Retrieved from the Internet: URL:http://www.genixtech.com/medical/GT%20 Rapid%20Tests%20inserts%20PDF/GT%20HCG%20r apid%20test%20inserts%20PDF/QuickStep%20Tu be%20Insert.pdf> [retrieved on 2007-03-07]
- OKU Y ET AL: "Development of oligonucleotide lateral-flow immunoassay for multi-parameter detection" JOURNAL OF IMMUNOLOGICAL METHODS, vol. 258, no. 1-2, 1 December 2001 (2001-12-01), pages 73-84, XP004311918
- SIMS P W ET AL: "IMMUNOPOLYMERASE CHAIN REACTION USING REAL-TIME POLYMERASE CHAIN REACTION FOR DETECTION" ANALYTICAL BIOCHEMISTRY, vol. 281, no. 2, 1 June 2000 (2000-06-01), pages 230-232, XP001147707
- SUGUNAN ET AL: "Heavy-metal ion sensors using chitosan-capped gold nanoparticles" SCIENCE AND TECHNOLOGY OF ADVANCED MATERIALS, vol. 6, no. 3-4, April 2005 (2005-04), pages 335-340, XP005039763
- HAZARIKA P. ET AL: 'Sensitive detection of proteins using difunctional DNA-gold nanoparticles' SMALL, WILEY - VCH VERLAG GMBH & CO. KGAA, DE LNKD- DOI:10.1002/SMLL.200500063 vol. 1, no. 8-9, 01 January 2005, pages 844 - 848, XP002407187 ISSN: 1613-6810

## Description

The present invention refers to a rapid immunochromatographic test device in the form of a test strip or a detection cup comprising a first and a second conjugate pad, wherein the first conjugate pad comprises a protein linked oligonucleotide and an antigen specific antibody, and the second conjugate pad comprises a protein linked complementary oligonucleotide, wherein both the protein linked oligonucleotides and the antibody are gold conjugated. The present invention further refers to a use of such test device for antigen detection in urine or saliva, e.g. human choriogonadotropin (hCG) in urine. The present invention also refers to a method for manufacturing such test device.

### Background of the Invention

In recent years, the *in vitro* diagnostics (IVD) industry has made enormous efforts to develop immunochromatographic tests. Such tests have found applications in both clinical and non-clinical fields ¹. A clinical utility of this test format has been shown for more than 150 different analytes, and many of them are target now of commercially available diagnostic products ³. The wide range of applications for such devices has been reviewed ^{1,2}.

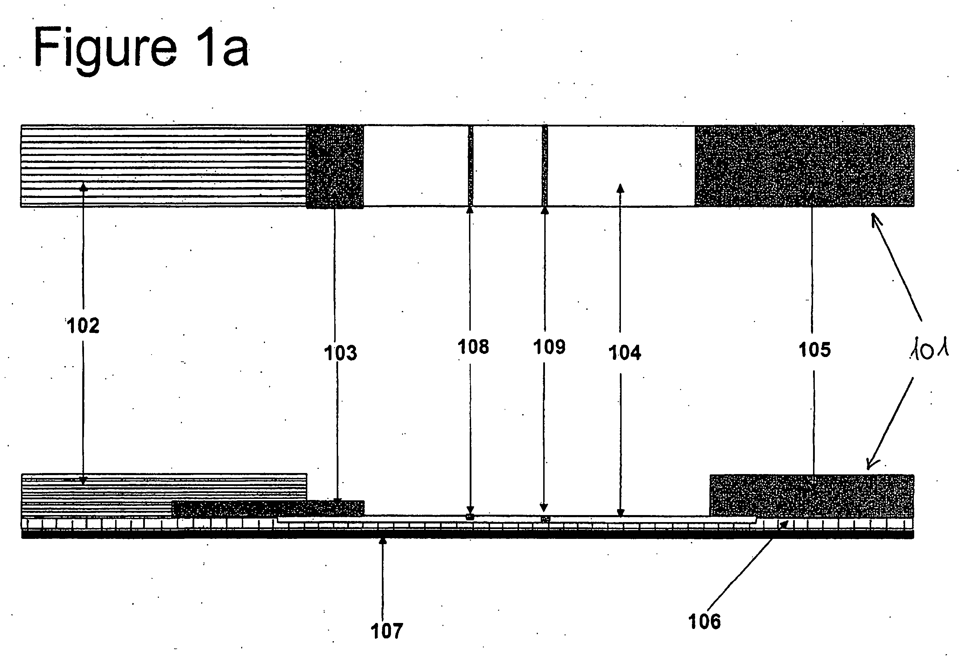

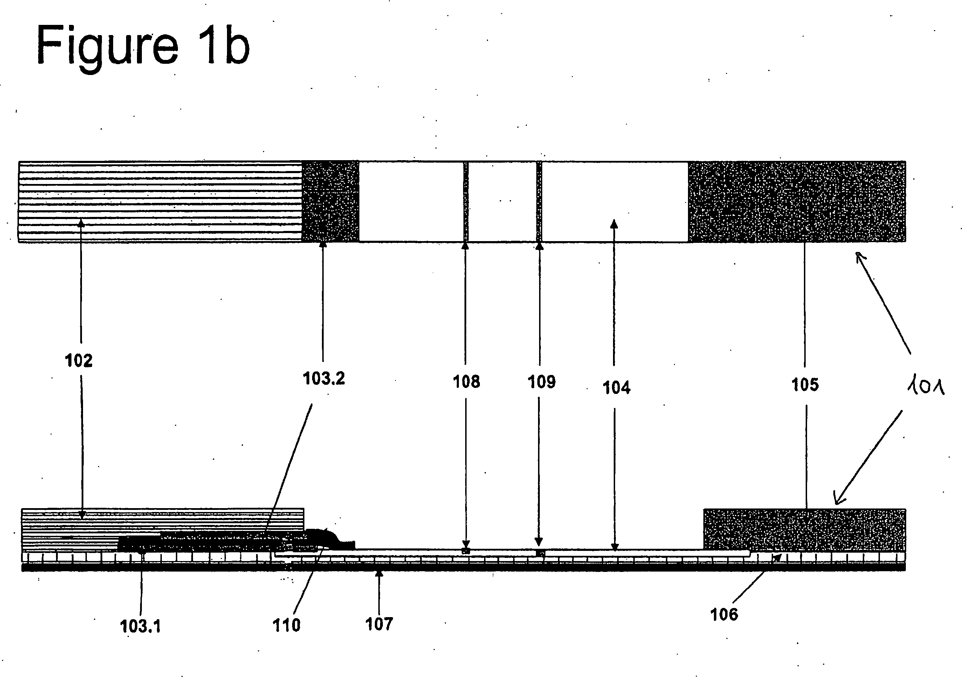

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

An example for such a rapid immunochromatographic test device is the "QuickStep HCG test". ⁵ This test is an immunoassay for early detection of pregnancy and comprises a test strip coated with goat anti-hCG and coloured colloidal gold-monoclonal mouse anti-hCG conjugate predried in a pad.

WO 02/46472 refers to the detection of analytes, in particular nucleic acids but also proteins. The method comprises contacting the nucleic acid with particles having oligonucleotides attached thereto.

Sugunan *et al.* (2005) discloses the use of gold nanoparticles capped with chitosan for sensing ions of heavy metals. ⁶. The polycationic nature of chitosan enables attachment of the polymer to the negatively charged gold nanoparticle surfaces through electrostatic interactions. Use of chitosan serves dual purpose of ensuring stability of the colloid and also to funtionalize the nanoparticles for use as sensors due to their optical properties.

Hazarika *et al.* (2005)⁷ refers to the sensitive detection of proteins using an alternative approach based on difunctional DNA-gold nanoparticles.

However, despite the wide use of rapid immunochromatographic test devices, their suitability is still limited with regard to certain applications. In particular, sensitivity remains a problem.

It is therefore an object of the present invention to provide a method and means for sensitive antigen detection in bodily fluids, e.g. urine and saliva.

### Summary of the Invention

The object of the present invention is solved by a rapid immunochromatographic test device for antigen detection, comprising a test strip with a sample zone, and further comprising at least a first and a second conjugate releasing pad, wherein the first conjugate pad comprises (i) a first conjugate of a colloidal gold conjugated protein linked to a first oligonucleotide and (ii) a colloidal gold conjugated antigen specific antibody, and the second conjugate pad comprises a second conjugate of a colloidal gold conjugated protein linked to a second oligonucleotide, which second oligonucleotide is complementary to the first oligonucleotide, wherein the first and the second conjugate pads are separated from each other such that the antigen to be detected is captured and thereby a complex is formed comprising said antigen and said first conjugate, then the complex will be captured from another site by an antigen specific antibody, wherein said antibody is immobilized within the sample zone, and then said second conjugate, which is released from said second conjugate pad also moves to the sample zone, where the first and second oligonucleotides bind to each other.

In one embodiment, the test device is in the form of a test strip.

In an alternative embodiment, the test device is in the form of a detection cup.

The first and the second conjugate pads are separated from each other. Such separation may be realized by a divider, preferably a plastic divider, sandwiched between the two pads, or by placing the two pads spaced apart from each other at different locations. The purpose of separating the two pads is to prevent untimely mixing of the conjugates comprised in the first and the second pad.

In one embodiment, the conjugate pads further comprise water-soluble chitosan which is optionally modified.

The object of the present invention is further solved by a use of a test device according to the present invention for the detection of an antigen in a sample of urine or saliva of a subject, preferably human.

In one embodiment, the antigen is urinary human choriogonadotropin (hCG).

The object of the present invention is further solved by a method for preparing a test device according to the present invention, comprising the following steps:
(a) providing a first conjugate solution comprising a colloidal gold conjugated protein linked to a first oligonucleotide and a colloidal gold conjugated antigen specific antibody, and applying said solution to a first conjugate pad;
(b) providing a second conjugate solution comprising a colloidal gold conjugated protein linked to a second oligonucleotide, which second oligonucleotide is complementary to the first oligonucleotide, and applying said solution to a second conjugate pad;
(c) providing a membrane comprising an antigen specific antibody immobilized within a sample zone and a non-specific antibody immobilized within a control zone. Immobilization can be realized by direct or indirect attachment to the membrane and has the effect that the sample zone antibody, in contrast to the antibody conjugate in the first conjugate pad, is not released.

In one embodiment, the method is for preparing a test device in the form of a test strip and additionally comprises the following steps:
(d) placing the membrane onto a backing material;
(e) placing the first conjugate pad onto the membrane;
(f) placing a divider, preferably a plastic divider, onto the first conjugate pad;
(g) placing the second conjugate pad onto the divider.

In an alternative embodiment, the method is for preparing a test device in the form of a detection cup and additionally comprises the following step:
(d') placing the membrane and the first and second conjugate pads into a container, wherein the first and second conjugate pads are separated from each other. Such separation may be realized by placing the two pads spaced apart from each other at different locations at the container interior.

In one embodiment of the method, water-soluble chitosan which is optionally modified is added during the preparation of the first and second conjugate solutions prior to conjugation of colloid gold with the antigen specific antibody and/or the protein linked oligonucleotides, i.e. the protein linked first oligonucleotide and the protein linked second oligonucleotide.

In a preferred embodiment of the method, the colloidal gold is prepared by reduction of a 1% aqueous solution of tetrachloroauric acid using trisodium citrate aqueous solution to produce spheroidal gold particles.

Thus, the present invention provides a rapid immunochromatographic detection system for antigen detection comprising a test strip and two conjugate releasing pads with different compositions. The first pad comprises an antibody specific for the antigen to be detected and a protein linked oligonucleotide ("sense" or first oligonucleotide). The antibody and the protein (e.g. bovine serum albumin, BSA) are conjugated with colloidal gold. The second conjugate pad comprises a further protein linked oligonucleotide ("antisense" or second oligonucleotide) also conjugated with colloidal gold. The both oligonucleotides, i.e. the sense and antisense oligonucleotide, are complementary to each other. When the antigen contained in the sample comes into contact with the antibody of the first pad, the antigen is captured by the antibody and thereby a complex is formed comprising the antigen to be detected, the conjugated antigen specific antibody and the conjugated protein linked sense oligonucleotide. This complex is carried to the sample zone where further antibody is immobilized. The complex will be captured then by the antigen specific antibody within the sample zone from another site. The conjugated protein linked antisense oligonucleotide which is released from the second conjugate pad also moves to the sample zone where the complementary oligonucleotides, i.e. the sense and the antisense oligonucleotides, bind to each other. Thus, the complex of antigen, antibody and the sense oligonucleotide which is bound within the sample zone serves as a target for the antisense oligonucleotide. Due to sense/antisense oligonucleotide and antigen/antibody interaction, a multi-complex is formed. The binding between the two conjugates will enhance the signal colour intensity and thus enhance the sensitivity of detection.

In addition to making use of the multi-complex formation, further sensitivity enhancement is achieved by using a large sample volume of urine or saliva and by using water-soluble chitosan (or modified water-soluble chitosan).

### Detailed Description of the Invention

### Brief Description of the Figures

- Figure 1a: shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test or sample zone 108, and a control zone 109.
- Figure 1b: shows top and side views of a rapid-flow immunochromatographic test device according to the present invention in the form of a test strip 101 including a sample pad 102, a first conjugate pad 103.1, a second conjugate pad 103.2, a membrane 104, an absorbent pad 105, an adhesive 106, a supporting backing 107, a test or sample zone 108, a control zone 109, and a conjugates divider 110 separating the conjugate pads 103.1 and 103.2.
- Figure 2a: shows an assembly comprising a test strip 201, an absorbent sample pad 202, a membrane 204, an absorbent pad 205, a sample zone 208 and a control zone 209.
- Figure 2b: shows an immunochromatographic detection cup comprising a sample-collecting container 210, a cap 211, a first conjugate pad 203 and a second conjugate pad 203', and the assembly shown in Fig. 2a.

### EXAMPLES

### EXAMPLE 1: Preparation of protein linked oligonucleotide

5 mg of bovine serum albumin (BSA) each was linked to an oligonucleotide (about 20 nucleotides having an amino group at the 5' terminus) and to a complementary oligonucleotide (about 20 nucleotides having an amino group at the 5' terminus) according to the method of Duncan *et al.* (1983)⁴ which can be illustrated as a procedure comprising the following steps:

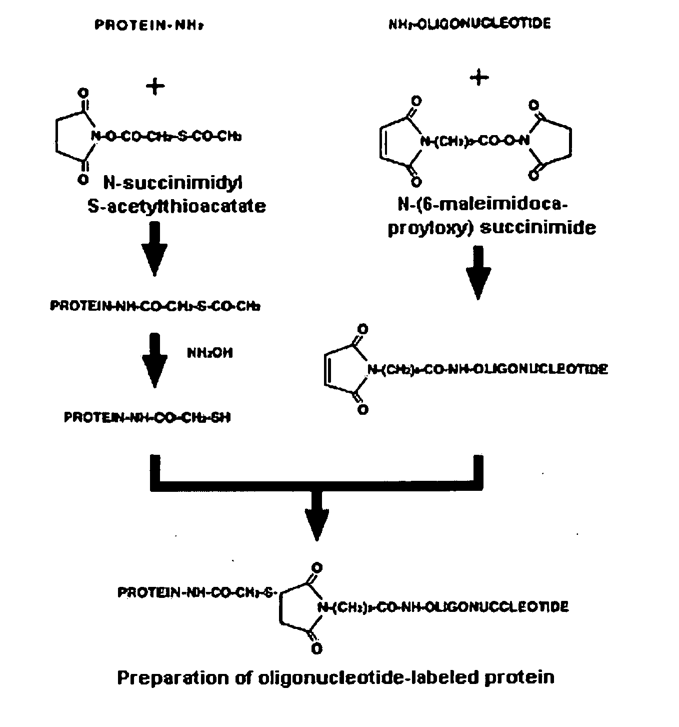

### EXAMPLE 2: Preparation of conjugate pads comprising protein linked oligonucleotide

The oligonucleotide and complementary oligonucleotide linked BSA prepared as described in Example 1 are further processed according to a procedure comprising the following steps:
(a) prepare oligonucleotide linked BSA solution (solution 1);
(b) prepare complementary oligonucleotide linked BSA solution (solution 2);
(c) prepare 1% aqueous solution of tetrachloroauric acid at room temperature;
(d) prepare 4% trisodium citrate aqueous solution at room temperature;
(e) prepare 0.05 M potassium carbonate aqueous solution at room temperature;
(f) prepare 400 ml of phosphate stabilizing buffer, pH 7.4, containing BSA, Tween 20, sucrose, polyvinylpyrrolidone and preservative (like sodium azide) at room temperature;
(g) prepare colloidal gold solution by reduction of 1.7 ml boiling tetrachloroauric acid solution (after dilution in 100 ml) using 1 ml trisodium citrate solution and equilibrate to room temperature;
(h) dilute the colloidal gold solution 1:1 using distilled water and adjust the pH at 7.4 using potassium carbonate solution at room temperature;
(i) prepare 200 ml of phosphate conjugation buffer, pH 7.4, at room temperature;
(j) partition the 200 ml conjugation buffer by dividing it between two flasks (100 ml each), i.e. a first and a second flask;
(k) add 0.5 mg of an aqueous antibody solution to the conjugation buffer in the first flask while stirring at room temperature;
(l) add 0.5 mg of the oligonucleotide linked BSA aqueous solution (solution 1) to the first flask at room temperature;
(m) add 1.0 mg of the complementary oligonucleotide linked BSA aqueous solution (solution 2) to the conjugation buffer in the second flask while stirring at room temperature;
(n) add 100 ml colloidal gold solution into each flask while stirring at room temperature;
(o) add 200 ml of stabilizing buffer to each flask and concentrate each conjugate solution by cooled (temperature around 15°C) high speed centrifugation (10,000 rpm for one hour);
(p) discard the supernatant and re-suspend the concentrated conjugates at room temperature;
(q) adjust the concentration for each of the two conjugates at O.D.₅₂₀ = 2.0;
(r) add 0.1 ml of Tween 20 to the first conjugate solution and soak a glass fibre sheet conjugate pad with the conjugate solution, then heat dry at temperature around 50°C;
(s) soak another glass fibre sheet conjugate pad with the second conjugate, then heat dry at a temperature around 50°C.

### EXAMPLE 3: Preparation of a test device in the form of a test strip

In case of a test strip, the first conjugate releasing pad 103.1 is prepared by soaking with oligonucleotide linked BSA and the antibody conjugate, while the other pad 103.2 is prepared by soaking with complementary oligonucleotide linked BSA conjugate (see Example 2).

In more detail, the test device is prepared according to a procedure comprising the following steps:
(a) prepare a phosphate sample buffer containing goat serum, ethylenediamine tetraacetic acid (EDTA), non-fat dry milk, preservative (like sodium azide) and Tween 20;
(b) soak a sample pad with the phosphate sample buffer and heat dry at a temperature around 50°C;
(c) prepare a colloidal gold solution (see Example 2);
(d) conjugate colloidal gold with oligonucleotide linked BSA and the antibody (e.g. antigen specific or non-specific antibody) to prepare the first conjugate (see Example 2), add Tween 20 to this first conjugate solution, soak a conjugate pad with this first conjugate solution and heat dry at a temperature around 50°C;
(e) conjugate colloidal gold with complementary oligonucleotide linked BSA to prepare the second conjugate, soak a conjugate pad with this second conjugate solution (see Example 2) and heat dry at a temperature around 50°C;
(f) print sample zone antibody (i.e. antigen specific antibody) and control zone antibody (i.e. non-specific antibody) onto a nitrocellulose membrane and heat dry at a temperature around 50°C;
(g) prepare a membrane blocking solution containing Tween 20 and non-fat dry milk;
(h) block the nitrocellulose membranes using the blocking solution and heat dry at a temperature around 50°C;
(i) laminate in the following order components of the test strip onto a backing material:
   (i.i) laminate the nitrocellulose membrane nearly in the middle of the test strip;
   (i.ii) laminate the absorbent pad at the end of the test strip (overlaps from the nitrocellulose membrane side);
   (i.iii) laminate the first conjugate pad on the other side of the nitrocellulose membrane;
   (i.iv) laminate the plastic divider onto the first conjugate pad (overlaps from the nitrocellulose membrane side);
   (i.v) laminate the second conjugate pad onto the divider (overlaps from the nitrocellulose membrane side);
   (i.vi) laminate the sample pad onto the other end of the sheet, the sample pad shall overlap with the two conjugate pads;
(j) pack the test strip into an aluminium pouch with a silica gel desiccant.

In steps (d) or (e), a sheet can be used instead of a single conjugate pad which is cut into several pads after drying. Similarly, in step (i), a sheet can be used which is cut into strips after having laminated all components.

The antibodies used may be polyclonal or monoclonal. Preferably, the antibody in the sample zone is identical to the antibody being part of the colloidal gold antibody conjugate comprised by the first conjugate pad. In case the specific antibody is from mouse, the control antibody is anti-mouse IgG, in case the specific antibody is from rabbit, the control antibody is anti-rabbit IgG.

Control and sample zone may be realized by lines. More than one sample zone and/or more than one control zone are also contemplated.

A urine wick can be used in case of urinary testing, while a blood filter is used in case of blood testing.

### EXAMPLE 4: Immunochromatographic detection cup

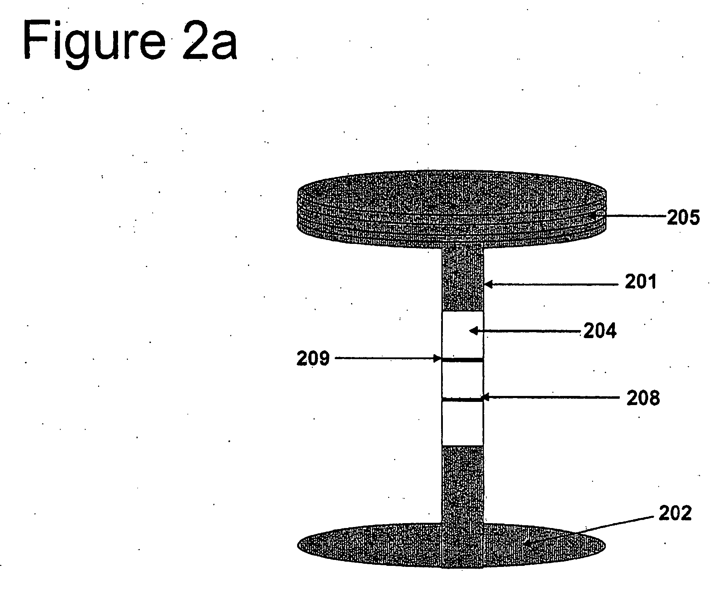

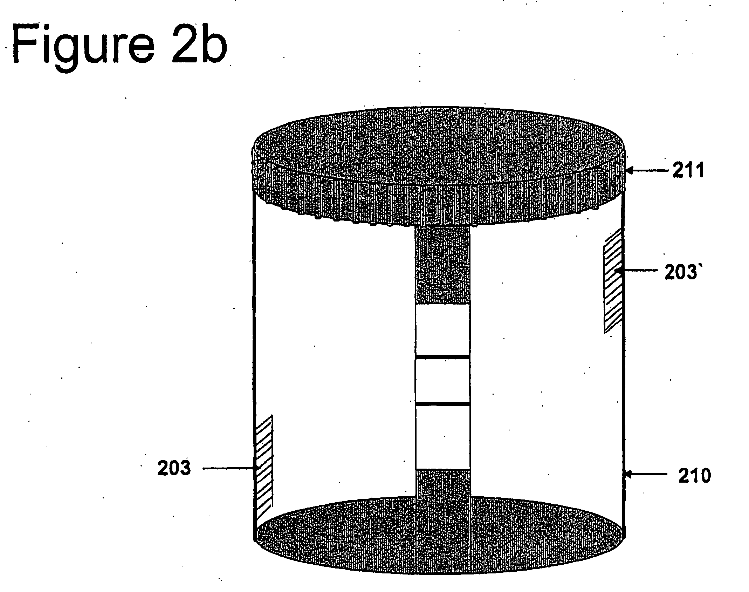

Another embodiment of the rapid immunochromatographic detection system of the present invention is shown in Fig. 2a, b. This immunochromatographic detection cup comprises a sample-collecting container 210, actually the "cup", a cap 211 for closing the container, and a detection test strip 201 inserted into the container 210. The test strip 201 may be placed inside the transparent container wall, and the test result can be read on the outer surface. The test strip 201 comprises a nitrocellulose membrane 204 and is linked via an absorbent pad 202 on the test strip 201 to an absorbent sample pad 202 placed on the bottom of the container 210. The absorbent sample pad 202 is designed to cover the inner surface of the container bottom. At the opposite end, the test strip 201 is linked via an absorbent pad 205 on the test strip to an absorbent pad 205 which is fitted into the cap 211 of the container and is designed to be thick enough to absorb more than 15 ml of sample.

A first gold conjugate releasing pad 203 is fixed to the inner surface of the container wall which pad 203 comprises a gold conjugated antibody and a gold conjugated BSA linked oligonucleotide. A second gold conjugate releasing pad 203' is also fixed to the inner surface of the container wall which pad 203' comprises a gold conjugated complementary oligonucleotide linked albumin wherein the complementary oligonucleotide is complementary to the oligonucleotide liked to BSA comprised by the first conjugate releasing pad 203. Importantly, both conjugate pads are fixed at different locations. The gold conjugates will begin releasing from pad 203 and 203' during the urine sample is streaming into the container 210. Release into the sample will increase the possibility of interaction between the conjugates and the antibodies to be detected.

The sample zone 208 also comprises a specific antibody. Non-specific antibody is immobilized within a control zone 209 onto the nitrocellulose membrane 204 for non-specific capturing of the gold conjugates. The antibodies used may be polyclonal or monoclonal. In the embodiment shown in Fig. 2a, b, the control zone 209 is realized by a control line. More than two sample zones 208 and/or more than one control zone 209 are also contemplated.

### EXAMPLE 5: Preparation of a test device in the form of a detection cup

In case of a urinary or saliva immunochromatographic detection cup, two pads are soaked each with one of the solutions containing gold conjugate, the first solution containing oligonucleotide linked BSA and an antigen specific antibody, the second solution containing complementary oligonucleotide linked BSA. The two different conjugate releasing pads 203 and 203' are fixed to the inner surface of the container 210 at different locations (see Fig. 2b).

In more detail, a detection cup is prepared according to a procedure comprising the following steps:
(a) prepare a phosphate sample buffer containing goat serum, ethylenediamine tetraacetic acid (EDTA), non-fat dry milk, preservative (like sodium azide) and Tween 20;
(b) soak a sample pad with the phosphate sample buffer and heat dry at a temperature around 50°C;
(c) prepare a colloidal gold solution (see Example 2);
(d) conjugate colloidal gold with oligonucleotide linked BSA and the antibody to prepare the first conjugate (see Example 2), add Tween 20 to this first conjugate solution, soak a conjugate pad with this first conjugate solution and heat dry at a temperature around 50°C;
(e) conjugate colloidal gold with complementary oligonucleotide linked BSA to prepare the second conjugate (see Example 2), soak a conjugate pad with this second conjugate solution and heat dry at a temperature around 50°C;
(f) print sample zone antibody and control zone antibody onto a nitrocellulose membrane and heat dry at a temperature around 50°C;
(g) prepare a membrane blocking solution containing Tween 20 and non fat dry milk;
(h) block the nitrocellulose membranes using the blocking solution and heat dry at a temperature around 50°C;
(i) laminate in the following order the components of a test strip to be inserted in the container onto a backing material sheet:
   (i.i) laminate the nitrocellulose membrane nearly in the middle of the test strip;
   (i.ii) laminate the absorbent pads at the end of the test strip (overlaps from the nitrocellulose membrane side);
(j) assemble the test strip into a plastic housing, i.e. the container;
(k) attach the first and the second conjugate pad inside the container;
(l) pack the detection cup into an aluminium pouch with a silica gel desiccant.

In steps (d) or (e), a sheet can be used instead of a single conjugate pad which is cut into several pads after drying. Similarly, in step (i), a sheet can be used which is cut into strips after having laminated all components.

The antibodies used may be polyclonal or monoclonal. Preferably, the antibody in the sample zone is identical to the antibody being part of the colloidal gold antibody conjugate comprised by the first conjugate pad. Control and sample zone may be realized by lines. More than one sample zone and/or more than one control zone are also contemplated.

A urine wick can be used in case of urinary testing, while a blood filter is used in case of blood testing.

### EXAMPLE 6: Pregnancy midstream test

In one embodiment, the detection cup is applicable for detecting of hCG in order to detect pregnancy. The first conjugate releasing pad 203 comprises gold conjugate 1, i.e. gold conjugated mouse anti-βhCG and conjugated BSA linked oligonucleotide 1. The second conjugate releasing pad 203' comprises gold conjugate 2, i.e. conjugated complementary oligonucleotide 2. The sample zone 208 comprises mouse anti-βhCG immobilized onto the nitrocellulose membrane 204. The control zone 209 comprises non-specific anti-mouse IgG. Sample and control zones 208, 209 turn into purple colour in case that hCG is present in the sample; only the control zone 209 turns into purple colour in case of hCG free sample.

### EXAMPLE 7: Amplification of gold conjugate colour intensity

The colloidal gold conjugate colour intensity was amplified using water-soluble chitosan (or modified water-soluble chitosan) as a colour intensity modification agent. The action of water-soluble chitosan (or modified water-soluble chitosan) in connection with the antibodies or antigens is on the colour intensity of the colloidal gold. Chitosan is added during the preparation of colloidal gold, but prior to the conjugation of colloidal gold with the proteins. Water-soluble chitosan (or modified water-soluble chitosan) affect the colour intensity of colloidal gold and so increases the ability of the human eye to identify the colour, and, thus, enables to detect very low concentrations of the analyte. Signal amplification lies in the range of up to 10folds. Colloidal gold could be prepared by the reduction of 1% aqueous solution of tetrachloroauric acid (HAuCl₄) using trisodium citrate aqueous solution to produce spheroidal gold particles. After colloidal gold preparation, water-soluble chitosan (or modified water-soluble chitosan) aqueous solution was added with a suitable volume and concentration to convert colour from purple to violet pending on the volume and concentration of the added modification solution.

### References

(1) J Chandler, N Robinson, and K Whiting, "Handling False Signals in Gold-Based Rapid Tests", IVD Technology 7, no. 2 (2001): 34-45; http://www.devicelink.com/ivdtarchive/01/03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3) TC Tisone et al., "Image Analysis for Rapid-Flow Diagnostics", IVD Technology 5, no. 5 (1999): 52-58; http://www.devicelink.com/ivdt/archive/99/09/010.html.
(4) Duncan, R.J.S., Weston, P.D., Wrigglesworth, R., 1983. A new reagent which may be used to introduce sulfhydryl groups into proteins, and its use in the preparation of conjugates for immunoassay. Anal. Biochem. 132, 68.
(5) "QuickStep™ HCG - One Step Urine Pregnancy Test", insert of a product of Innovatek Medical Inc., CA, March 1998, also published on the internet.
(6) A Sugunan, C Thanachayanont, J Dutta, JG Hilborn, "Heavy-metal ion sensors using chitosan-capped gold nanoparticles", Science and Technology of Advanced Material 6 (2005): 335-340.
(7) P Hazarika, B Ceyhan, CM Niemeyer, "Sensitive detection of proteins using difunctional DNA-gold nanoparticles", Small 1 (2005): 844-848.

## Claims

1. A rapid immunochromatographic test device for antigen detection, comprising a test strip with a sample zone, and further comprising at least a first and a second conjugate releasing pad, wherein the first conjugate pad comprises a first conjugate of (i) a colloidal gold conjugated protein linked to a first oligonucleotide and (ii) a colloidal gold conjugated antigen specific antibody, and the second conjugate pad comprises a second conjugate of a colloidal gold conjugated protein linked to a second oligonucleotide, which second oligonucleotide is complementary to the first oligonucleotide, wherein the first and the second conjugate pads are separated from each other such that the antigen to be detected is captured and thereby a complex is formed comprising said antigen and said first conjugate, then the complex will be captured from another site by an antigen specific antibody, wherein said antibody is immobilized within the sample zone, and then said second conjugate, which is released from said second conjugate pad also moves to the sample zone, where the first and second oligonucleotides bind to each other.

2. The test device according to claim 1 in the form of a test strip.

3. The test device according to claim 1 in the form of a detection cup.

4. The test device according to any of the preceding claims, wherein the colloidal gold on the conjugate pads further comprises water-soluble chitosan which is optionally modified.

5. A use of a test device according to any of claims 1 to 4 for the detection of an antigen in a sample of urine or saliva of a subject, preferably human.

6. The use according to claim 5, wherein the antigen is urinary hCG

7. A method for preparing a test device according to any of claims 1 to 4, comprising the following steps:
(a) providing a first conjugate solution comprising a colloidal gold conjugated protein linked to a first oligonucleotide and a colloidal gold conjugated antibody, and applying said solution to a first conjugate pad;
(b) providing a second conjugate solution comprising a colloidal gold conjugated protein linked to a second oligonucleotide, and applying said solution to a second conjugate pad;
(c) providing a membrane comprising an antigen specific antibody immobilized within a sample zone and a non-specific antibody immobilized within a control zone.

8. The method according to claim 7 for preparing a test device in the form of a test strip, additionally comprising the following steps:
(d) placing the membrane onto a backing material;
(e) placing the first conjugate pad onto the membrane;
(f) placing a divider, preferably a plastic divider, onto the first conjugate pad;
(g) placing the second conjugate pad onto the divider.

9. The method according to claim 7 for preparing a test device in the form of a detection cup, additionally comprising the following step:
(d') placing the membrane and the first and second conjugate pads into a container, wherein the first and second conjugate pads are separated from each other.

10. The method according to any of claims 7 to 9, wherein water-soluble chitosan which is optionally modified is added during the preparation of colloidal gold for the provision of the first and second conjugate solutions, but prior to the conjugation of colloidal gold with the antibody and/or protein linked oligonucleotide.

11. The method according to claim 10, wherein the colloidal gold is prepared by reduction of a 1% aqueous solution of tetrachloroauric acid using trisodium citrate aqueous solution to produce spheroidal gold particles.

## Patentansprüche

1. Immunochromatographische Schnelltestvorrichtung zur Antigen-Detektion, umfassend einen Teststreifen mit einer Probenzone und weiter umfassend wenigstens ein erstes und ein zweites Konjugatfreisetzungskissen, wobei das erste Konjugatkissen ein erstes Konjugat aus (i) einem mit kolloidalem Gold konjugierten Protein, das mit einem ersten Oligonukleotid verknüpft ist, und (ii) einem mit kolloidalem Gold konjugierten antigenspezifischen Antikörper umfasst und das zweite Konjugatkissen ein zweites Konjugat aus einem mit kolloidalem Gold konjugierten Protein, das mit einem zweiten Oligonukleotid verknüpft ist, umfasst, wobei das zweite Oligonukleotid komplementär zum ersten Oligonukleotid ist, wobei die ersten und zweiten Konjugatkissen voneinander getrennt sind, so dass das zu detektierende Antigen eingefangen wird und dadurch ein Komplex gebildet wird, der das Antigen und das erste Konjugat umfasst, dann der Komplex von einer anderen Stelle durch einen antigenspezifischen Antikörper eingefangen werden wird, wobei der Antikörper in der Probenzone immobilisiert ist, und dann das zweite Konjugat, das aus dem zweiten Konjugatkissen freigesetzt wird, ebenfalls zur Probenzone wandert, wo die ersten und zweiten Oligonukleotide aneinander binden.

2. Testvorrichtung nach Anspruch 1 in der Form eines Teststreifens.

3. Testvorrichtung nach Anspruch 1 in der Form einer Detektionsschale.

4. Testvorrichtung nach einem der vorangehenden Ansprüche, wobei das kolloidale Gold auf den Konjugatkissen weiter wasserlösliches Chitosan umfasst, das gegebenenfalls modifiziert ist.

5. Verwendung einer Testvorrichtung nach einem der Ansprüche 1 bis 4 zur Detektion eines Antigens in einer Harn- oder Speichelprobe eines Patienten, vorzugsweise Menschen.

6. Verwendung nach Anspruch 5, wobei das Antigen Harn-hCG ist.

7. Verfahren zur Herstellung einer Testvorrichtung nach einem der Ansprüche 1 bis 4, umfassend die folgenden Schritte:
(a) Bereitstellen einer ersten Konjugatlösung, die ein erstes mit kolloidalem Gold konjugiertes Protein, das mit einem ersten Oligonukleotid verknüpft ist, und einen mit kolloidalem Gold konjugierten Antikörper umfasst, und Aufbringen der Lösung auf ein erstes Konjugatkissen;
(b) Bereitstellen einer zweiten Konjugatlösung, die ein mit kolloidalem Gold konjugiertes Protein, das mit einem zweiten Oligonukleotid verknüpft ist, umfasst, und Aufbringen der Lösung auf ein zweites Konjugatkissen;
(c) Bereitstellen einer Membran, die einen antigenspezifischen Antikörper, der in einer Probenzone immobilisiert ist, und einen nicht-spezifischen Antikörper, der in einer Kontrollzone immobilisiert ist, umfasst.

8. Verfahren nach Anspruch 7 zur Herstellung einer Testvorrichtung in der Form eines Teststreifens, das zusätzlich die folgenden Schritte umfasst:
(d) Aufbringen der Membran auf ein Rückschichtmaterial;
(e) Aufbringen des ersten Konjugatkissens auf die Membran;
(f) Aufbringen eines Teilers, vorzugsweise eines Kunststoffteilers, auf das erste Konjugatkissen;
(g) Aufbringen des zweiten Konjugatkissens auf den Teiler.

9. Verfahren nach Anspruch 7 zur Herstellung einer Testvorrichtung in der Form einer Detektionsschale, das zusätzlich den folgenden Schritt umfasst:
(d') Einbringen der Membran und der ersten und zweiten Konjugatkissen in einen Behälter, wobei die ersten und zweiten Konjugatkissen voneinander getrennt sind.

10. Verfahren nach einem der Ansprüche 7 bis 9, wobei wasserlösliches Chitosan, das gegebenenfalls modifiziert ist, während der Herstellung von kolloidalem Gold für die Bereitstellung der ersten und zweiten Konjugatlösungen zugegeben wird, aber vor der Konjugation von kolloidalem Gold mit dem mit Antikörper und/oder Protein verknüpften Oligonukleotid.

11. Verfahren nach Anspruch 10, wobei das kolloidale Gold hergestellt wird durch Reduktion einer 1%-igen wässrigen Lösung von Tetrachlorgoldsäure durch Verwendung von wässriger Trinatriumcitratlösung, um sphäroidale Goldteilchen herzustellen.

## Revendications

1. Dispositif d'essai rapide immunochromatographique pour la détection d'antigènes, comprenant une bande d'essai avec une zone à échantillon, et comprenant en outre au moins un tampon de libération d'un premier et d'un deuxième conjugués, dans lequel le tampon de premier conjugué comprend un premier conjugué (i) d'une protéine conjuguée à l'or colloïdal liée à un premier oligonucléotide et (ii) un anticorps spécifique de l'antigène conjugué à l'or colloïdal, et le tampon de deuxième conjugué comprend un deuxième conjugué d'une protéine conjuguée à l'or colloïdal liée à un deuxième oligonucléotide, lequel deuxième oligonucléotide est complémentaire au premier oligonucléotide, dans lequel les tampons des premier et deuxième conjugués sont séparés l'un de l'autre de sorte que l'antigène à détecter soit capturé et ainsi un complexe est formé comprenant ledit antigène et ledit premier conjugué, ensuite le complexe sera capturé à partir d'un autre site par un anticorps spécifique de l'antigène, dans lequel ledit anticorps est immobilisé dans la zone à échantillon, et ensuite ledit deuxième conjugué, qui est libéré dudit tampon de deuxième conjugué se déplace également vers la zone à échantillon, où les premier et deuxième oligonucléotides se lient entre eux.

2. Dispositif d'essai selon la revendication 1 sous forme d'une bande d'essai.

3. Dispositif d'essai selon la revendication 1 sous forme d'une coupe de détection.

4. Dispositif d'essai selon l'une des revendications précédentes, dans lequel l'or colloïdal sur les tampons de conjugués comprend en outre du chitosane soluble dans l'eau qui est éventuellement modifié.

5. Utilisation d'un dispositif d'essai selon l'une des revendications 1 à 4 pour la détection d'un antigène dans un échantillon d'urine ou de salive d'un sujet, de préférence humain.

6. Utilisation selon la revendication 5, dans laquelle l'antigène est une hCG de l'urine.

7. Procédé de préparation d'un dispositif d'essai selon l'une des revendications 1 à 4, comprenant les étapes suivantes qui consistent à :
(a) fournir une première solution de conjugués comprenant une protéine conjuguée à l'or colloïdal liée à un premier oligonucléotide et un anticorps conjugué à l'or colloïdal, et appliquer ladite solution à un premier tampon conjugué ;
(b) fournir une deuxième solution de conjugués comprenant une protéine conjuguée à l'or colloïdal liée à un deuxième oligonucléotide, et appliquer ladite solution à un deuxième tampon conjugué ;
(c) fournir une membrane comprenant un anticorps spécifique de l'antigène immobilisé dans une zone à échantillon et un anticorps non spécifique immobilisé dans une zone de contrôle.

8. Procédé selon la revendication 7 pour la préparation d'un dispositif d'essai sous forme d'une bande d'essai, comprenant en outre les étapes suivantes qui consistent à :
(d) placer la membrane sur un matériau de support ;
(e) placer le premier tampon conjugué sur la membrane ;
(f) placer un séparateur, de préférence un séparateur plastique, sur le premier tampon conjugué ;
(g) placer le deuxième tampon conjugué sur le séparateur.

9. Procédé selon la revendication 7 pour la préparation d'un dispositif d'essai sous forme d'une coupe de détection, comprenant en outre l'étape suivante qui consiste à :
(d') placer la membrane et les premier et deuxième tampons conjugués dans un flacon, dans lequel les premier et deuxième tampons conjugués sont séparés l'un de l'autre.

10. Procédé selon l'une des revendications 7 à 9, dans lequel du chitosane soluble dans l'eau qui est éventuellement modifié est ajouté durant la préparation de l'or colloïdal pour fournir les première et deuxième solutions de conjugués, mais avant la conjugaison de l'or colloïdal avec l'anticorps et/ou la protéine liée à l'oligonucléotide.

11. Procédé selon la revendication 10, dans lequel l'or colloïdal est préparé par la réduction de 1% d'une solution aqueuse d'acide tétrachloroaurique en utilisant une solution aqueuse de citrate trisodique pour produire des particules d'or sphéroïdales.
